(19) **Europäisches Patentamt**
**European Patent Office**
**Office européen des brevets**

(11) **EP 2 995 248 A1**

(12) **EUROPEAN PATENT APPLICATION**
published in accordance with Art. 153(4) EPC

(43) Date of publication:
**16.03.2016 Bulletin 2016/11**

(21) Application number: **13857574.1**

(22) Date of filing: **02.12.2013**

(51) Int Cl.:
*A61B 5/02* (2006.01)    *A61B 5/04* (2006.01)
*A61B 5/145* (2006.01)    *A61B 10/00* (2006.01)
*G06F 19/00* (2011.01)

(86) International application number:
**PCT/RU2013/001083**

(87) International publication number:
**WO 2014/081348 (30.05.2014 Gazette 2014/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2012 RU 2012150123**

(71) Applicant: **Cardiobionica SAGL**
**6900 Lugano (CH)**

(72) Inventors:
• **OSTROZHINSKIJ, Vladimir Aleksandrovich**
**Moscow 121108 (RU)**
• **GURFINKEL, Yury Il'ich**
**Moscow 119602 (RU)**

(74) Representative: **Rapisardi, Mariacristina**
**Ufficio Brevetti Rapisardi S.r.l.**
**Via Serbelloni, 12**
**20122 Milano (IT)**

(54) **METHOD AND DEVICE FOR ASSESSING THE RISK OF CARDIOVASCULAR COMPLICATIONS**

(57) The group of inventions is directed to improving the reliability and objectivity of assessment of the risk of cardiovascular complications. Using optical capillaroscopy of the nail fold of a finger in a state of reactive hyperemia and in the absence thereof, the size of the perivascular area and the diameters of the venous and arterial portions of the capillaries are measured, pulse wave velocity and the level of endothelial function in the upper limb are measured simultaneously relative to the R peak of an electrocardiogram, and an index K of the risk of cardiovascular complications is calculated using the given expression and categorized. The device comprises: a pneumatic means for creating occlusion in a limb of the patient and pressure sensors connected to occlusion cuffs; an input unit capable of receiving and regulating the level of signals from the output of the pressure sensors and of amplifying and pre-filtering said signals; an optical capillaroscopy unit capable of measuring the size of the perivascular zone and the diameters of the venous and arterial portions of the nail fold capillaries of a finger in a state of reactive hyperemia and in the absence thereof; an electrocardiogram recording unit capable of generating a pulsed synchronization signal based on the R wave of the electrocardiogram; a unit for amplitude-to-digital conversion, digital signal processing and control, a switching unit, and a unit for communicating with a computer.

FIG. 1

**Description**

Technical field

This group of inventions relates to medical equipment and may be used to determine and predict the condition of the cardiovascular system.

Background art

Cardiovascular disease (CVD), associated with arteriosclerosis, is a major cause of adult mortality in both economically developed and developing countries. One reason for high mortality from CVD is the lack of effective measures for primary and secondary prevention of cardiovascular complications, which ensure timely detection and correction of risk factors.

The concept of total cardiovascular risk was developed and implemented in general practice during the 1990s. Total cardiovascular risk, a term which has become commonplace in today's current clinical practice, designates a combination of various factors indicating the level of predicted risk of developing fatal and non-fatal cardiovascular complications, expressed as percentages. This fully applies to patients with type 2 diabetes mellitus (DM), including complications by arterial hypertension, since the incidence of the disease is steadily on the rise both in Russia and all over the world. The course and prognosis of diabetes mellitus is largely determined by the degree of impairment of macro and microcirculation (MC) vessels. The vascular endothelium is a wall, which serves as a kind of barrier in terms of ongoing adverse effects of hemodynamic and metabolic abnormalities in case of diabetes mellitus. It is obvious that timely diagnosis makes it possible to detect and quantify the degree of risk associated with cardiovascular complications, ascertain the proper treatment and monitor it using the proposed method over time.

There are several methods that can be used to assess total cardiovascular risk, such as the Framingham Risk Score, the PROCRAM mathematical model in the form of a CERCA computer program, and the European SCORE model, which are divided into four risk categories. LOW RISK CATEGORY. It includes: 1) younger patients without clinical manifestations of CVD, with one moderately expressed risk factor (RF); 2) patients with a negative family history: the closest relatives displaying the early onset of CVD (men < 55 years of age, women < 65 years of age).

INTERMEDIATE RISK CATEGORY. It includes patients with no clinical manifestations of CVD, but who are at risk of developing vascular arteriosclerosis due to the presence of more than two RFs even if the assessment scale SCORE 10-year risk is less than 5%; a marked increase of one RF - total cholesterol $\geq$ 8 mmol/l (320 mg/dL), LDL cholesterol $\geq$ 6 mmol/l (240 mg/dL), BP $\geq$ 180/110 mm Hg.

HIGH AND VERY HIGH RISK CATEGORY. These groups include 1) patients with any clinical manifestations of CHD, peripheral artery disease, cerebral arteriosclerosis, or abdominal aortic aneurysm; 2) patients without clinical implications of the above-mentioned diseases, but having several risk factors and with a fatal risk reading above 5% on the 10-year SCORE scale; 3) patients with types 1 and 2 DM, especially those with microalbuminuria. This category of patients requires close medical supervision, since the risk of developing fatal or nonfatal cardiovascular complications is quite high over the next few years.

As can be seen from the foregoing, these risk category assessment methods are not precise, and frequently require the determination of additional risk factors, yield strictly qualitative features of the degree of risk associated with cardiovascular disease, and do not provide a quantitative assessment.

There are some well-known methods for assessing the risk of cardiovascular complications based on additional research. In the invention (RU2410017 C1, Khoronenko et al., 27.01.2011), the individual risk index for the development of perioperative cardiovascular complications during extracardiac surgery is calculated according to a formula that includes the age of the patient, symptoms of chronic heart failure, angina of class II and III functional classification, ectopic heartbeat, and frequent ventricular arrhythmia during normal heart rhythm. The patent (RU2342068 C2, OMRON HEALTHCARE CO., LTD, (JP), 27.12.2008 describes an electronic blood pressure monitor that makes it possible to perform a quantitative assessment associated with CVD risk. In accordance with the main program executed by the central processing unit (CPU), a collection of data samples from the measurement results obtained during the prescribed period is retrieved from the BP readings stored in the memory. The invention (RU2405418 C1, Podtaev et al., 10.12.2010) relates to the diagnosis of endothelial dysfunction in patients with diabetes through cold test and thermometry of the nail phalanx of the index finger.

However, the above methods do not take into account the parameters of capillaries of perivascular tissues, or such macro-circulation parameters as the propagation of pulse wave velocity (PPWV) and BP, without which the conclusion on vascular tone and the condition of peripheral arteries would not be complete. These indicators and particularly the endothelial function not only reflect the degree of relaxation of the smooth muscles of the vessel, but also the inhibition of adhesion and platelet clumping, as well as macrophage activity.

Using a non-invasive method to determine morphological and dynamic characteristics of capillaries and capillary blood flow (RU 2389434 C2, Gurfinkel et al., New Energy Technologies LLC, 20.05.2010), areas of research are selected as a result of examining the entire perivascular area of the nail bed. This process provides a set of frames under the sequential convection of the radiation sources of the lighting system located around the bed, while monitoring the dynamic state of blood flow is implemented at a speed of at least 100 fps.

The closest method to that of the patent is a method for assessing the risk of cardiovascular diseases endemic to the Russian population (RU2352258 C2, Oganov, et al., 20.04.2009 is the closest analogue to the first invention of the group). A regression model is built in proportion to the risk, with the following risk factors being used: the age of the individual, systolic blood pressure, heart rate, the relative weight and body mass index, the status of exertional angina and typical myocardial infarction pain, as well as a resting electrocardiogram. The absolute risk of developing cardiovascular disease is then calculated using the original formula.

However, this method does not take into account macro and microcirculation indicators, without which the risk profile is not complete, as the course and prognosis of diabetes mellitus is largely determined by the degree of occlusion of macro and microcirculation. Changes in the microcirculation system are detected already at the stage of impaired glucose tolerance and are of a generalized nature.

A description is given of devices that make it possible to a certain extent to record the status and predict the condition of the cardiovascular system based on some of the above parameters, containing a number of units that combine monitoring and computing functions.

The PPWV parameter is known to characterize the elastic tension of the vascular walls, being one of the most reliable indicators of their visco-elastic status. A device used to determine the state of the cardiovascular system (RU2343826 C1, AMDT LLC, Gurfinkel et al., 20.01.2009) contains a compressor, air valve control, two occlusive cuffs, one of which is a shoulder cuff, the other a forearm cuff, pressure sensors and pulse wave output filters, and analog-to-digital converter and a control unit connected to the computer. The cuffs are designed to be attached to one of the patient's arms and are connected through an adjustable air valve. The control unit contains a microprocessor, a switch and a unit that is connected to a computer.

The patent (RU 2373846 C1, Pegov et al., 27.11.2009) describes a device for long-term non-invasive monitoring of the parameters of microcirculation in capillaries and the spectral characteristics of blood and the surrounding tissues. The device comprises an optical observation system with lighting units and image detectors connected to an electronic signal processing unit, and attached to the chassis for placement of the object of study in such a way that the external part with the nail faces the visual window of the optical observation system. The device also includes at least two image detectors. The invention (US6939304 B2, SCHNALL ROBERT et al., 08.09. 2005) describes a method and apparatus for non-invasive determination of endothelial dysfunction, which involves preliminary clamping of a certain part of the patient's arm or leg. During the test, the patient's finger or toe is monitored using a finger probe to evaluate the endothelial activity after recovery of blood flow in the hand that was clamped. A reference probe is located on the finger of the other hand that was not clamped, making it possible to compensate for the shifts inherent in vascular beds.

Another invention (RU2294689 C2, Walejko et al., 10.03.2007) describes an adaptive optical system of the observation device with lighting and an image receiver connected to an electronic signal processing unit, and attached to the cradle structure. The finger support is designed to attach the finger to the outer and inner side through clamping force which is directed perpendicular to the longitudinal axis of the cradle structure. The focusing axis of the adaptive optical observation system is designed to move automatically in three mutually perpendicular directions.

The application (WO9804182 (A2), GOOR DANIEL A et al., 05.02.1998) describes a method and device used to indicate a state or conditions, for example in the event of myocardial ischemia or sleep apnoea, by determining the reaction of peripheral arterial tone in response to a change in status or conditions. Sufficient pressure is applied around the distal part of the subject's finger to prevent uncontrolled venous outflow and to partially relieve wall tension, while not fully pinching the artery of that distal segment. The apparatus includes the patient's finger probe in the shape of a tube, and which is in contact with the cuff. The probe is connected to the sensor/transducer of monitored changes in vascular tone. In the application (US2006224073 (A1), LIN, KANG-PING et al., 05.10.2006), a device is described for the diagnosis of cardiovascular diseases on the basis of ECG signals using multiple processing methods to obtain ECG parameters, the vascular stiffness index (SI), the vascular reflection index (RI), and pulse wave velocity (PWV). The closest analogue of the second invention of the group is a computer device for determining the state of the cardiovascular system by recording mean arterial pressure and vascular pressure, including PPWV, synchronized with the QRS unit and the ST segment of the ECG (US2006264771 (A1), LIN KANG-PING et.al., 23.11.2006) - the closest analogue to the second invention of the group). The device includes an occlusive cuff, connected to the compressor and an electro-magnetic valve, which are controlled by computer via signals from the signal processing input module. The input module is connected to the ECG unit by two electrodes and a pressure sensor. The input module contains analog processing and analog-to-digital conversion units.

Disadvantages include the inability to assess the condition of the cardiovascular system under such an important component as microcirculation, and in particular, the parameters of capillary blood flow and the surrounding capillary tissues where gas exchange and mass transfer, the most significant processes in terms of physiology, are carried out.

In addition, the use of a single occlusive cuff reduces noise measurements and limits the accuracy of waveform analysis.

Disclosure of the invention

The patent-pending method for assessing the risk of cardiovascular complications includes the following operations:

- determining the size of the perivascular zone, the diameters of venous and arterial areas of capillaries through optical-capillaroscopy of the nail fold of the finger;
- determining values of pulse wave velocity propagation and the endothelial function in the upper limb synchronously relative to the R peak of the ECG, and blood pressure measurement;
- calculating the K risk index of cardiovascular complications according to the formula:

$$K = (PZ + R + PWV + E + BP) \cdot 100 \ (1)$$

- assigning risk of cardiovascular complications when the K index is in the range of 1-249 to the low risk category, when the K index is in the range of 250-499 to the intermediate risk category, when the K index is in the range of 500-749 to the high risk category, and when the K index is in the range of 750-1000 to the very high risk category.

A difference in the method which is the closest analogue is that it uses a different calculation formula, and accordingly records other parameters (dimensions of the perivascular area, venous and arterial areas of capillaries, propagation of the pulse wave velocity and the endothelial function), while the only parameter which is totally recorded is BP.

The method is characterized in that the current value of the R* capillary remodelling coefficient is determined using the formula R = dv/da, where dv and da are the diameters of the venous and the arterial areas of the capillaries, $\mu$m, respectively.

The method can also be characterized in that the current PZ* value for the size of the perivascular area is determined as the distance between the most remote point of the perivascular area and the nearest transition point of the capillary area.

The method can also be characterized by the fact that PPWV is determined in the shoulder-wrist area using a sensor to record pressure signals which correspond to the time the pulse wave takes to pass when there is an occlusion, with subsequent averaging and accumulation of the above-mentioned signals during a specific time interval of measurements as well as calculation of the current PWV value for the specified area using the formula: PWV*= L/$\Delta$T mps, where L and $\Delta$T are the distance and signal latency between the pressure sensors, respectively.

In addition, the method may be characterized by the fact that the current value of the E* endothelial function is determined on the basis of results of measuring the amplitude of U1, U2 pressure sensor signals in the same area of the shoulder-wrist, at least twice, before and after the test is taken with compression of the brachial artery, preferably at three-minute intervals, averaging and accumulation of signals during the specified time interval of measurements before and after the test with compression of the measurements and calculation of the index using the formula E*= Umax2/Umax1, where Umax1 and Umax2 are the maximum values of the amplitudes of the pressure sensor signals before and after the test, respectively.

The patent-pending device for assessing the risk of cardiovascular complications includes pneumatic facilities to create occlusion on a limb of the subject, containing occlusive cuffs connected through an electro-pneumatic valve compressor and an electro-pneumatic valve with smooth deflation; pressure sensors connected to the occlusive cuffs; the input unit has the ability to receive and modulate the level of the output signal from the pressure sensor, signal intensity and pre-filtering;

the optical-capillaroscopy unit has the ability to determine the size of the perivascular zone, the diameters of venous and arterial areas of nailfold capillaries of the finger in terms of reactive hyperaemia or the absence thereof;

the electrocardiogram recorder has the ability to generate a pulse synchronization signal on the R wave of the electrocardiogram;

the amplitude-to-digital conversion, digital signal processing and control unit, the input of which is connected to the output of the above-mentioned input unit and synchronization output of the above-mentioned electrocardiogram recorder, which is capable of averaging and accumulating signals over the specified time interval of measurements and determination of their amplitude and time parameters;

the switching unit, the input of which is connected to the output of the above-mentioned digital amplitude converter, digital processing and control of signals, while the output is connected to the control inputs of the above-mentioned electro-pneumatic valves and compressor;

the computer interface, the inputs and outputs of which are connected to the amplitude-to-digital conversion, digital signal processing and control unit, and the computer, whereas the inputs are connected to the outputs of the electrocardiogram recorder and the optical-capillaroscopy unit, the synchronization input of which is connected to the synchronization output of the above-mentioned electrocardiogram recorder.

A difference compared to the device regarded as the closest analogue is the presence of a capillaroscopy unit, which has a system controlling three (not one) cuffs, thus making it possible to measure PPWV and the index of the endothelial function during a single research stage. A different algorithm is used to operate the amplitude-to-digital conversion unit, as well as in digital processing and control of signals.

The device may be characterized in that the input unit contains four digital potentiometers, whose signal inputs are unit inputs, and five signal amplifiers are equipped with band pass filters, the outputs of which are the outputs of the unit. The digital outputs of the potentiometers are connected to the inputs of the four amplifiers in series, and the fifth amplifier input is connected directly to the input of one of the digital potentiometers.

The device can be characterized in that the video capillaroscopy unit contains a cradle to brush the upper limb, made of heat-conducting material connected to the cradle tool for highlighting areas of the nail plate of the finger extremity, an occlusive finger cuff, an optical sensor, a tool for maintaining the temperature of the heated bed, an optical sensor connected to a microscope with a video camera, a microcontroller linked to a video camera, which is the output of the optical capillaroscopy unit, 3D positioning of the cradle relative to the optical sensor, configured to adjust the image area of the nail plate.

The device is also characterized in that the cradle performs the function of an electrode of the ECG recorder.

The device is also characterized in that a microcontroller contains a frame counter image assigning frame sequential numbers with the option to reset the counter of the synchronization signal on the R wave of the electrocardiogram.

The technical result of the method is to increase the reliability and objectivity of the risk assessment of cardiovascular complications through one phase of the study using the new metric. The technical result of the device consists in providing a joint measurement of PPWV and the endothelial function of the arteries, and also the condition of the capillaries, including remodelling diagnostics in the course of the pathological process.

The basis of the present invention consists of theoretical and experimental studies of authors who have shown that it is possible to objectify the assessment required in pathology by taking into account additional parameters of the cardiovascular system. These include changing the state of the capillaries, their remodelling in the case of pathology, as has been observed already in the early stages of hypertension (see Gurfinkel Yu.I., Makeeva, O.V., Ostrozhinsky V.A. Features of microcirculation, the endothelial function, and the propagation of pulse wave velocity in patients with initial stages of hypertension// Functional diagnostics. 2010. No. 2, pp. 18-25). In addition, a quantitative assessment of the degree of swelling in pericapillary tissues also characterizes symptoms of chronic heart failure (see Gurfinkel Yu.I., Katz N.V., Parfenova L.M., Ivanova I.Yu., and Orlov V.A. Comparative study of the propagation velocity of the pulse wave and endothelial function in healthy individuals and patients with cardiovascular pathology/Russian Journal of Cardiology. 2009, No. 2, pp. 38-43; Gurfinkel Yu.I., Kudutkina M.L, Parfenova L.M., Orlova V.A. Features of microcirculation in patients with chronic heart failure against the background of treatment with ACE inhibitors and diuretics/Russian Journal of Cardiology. 2011, No. 2, pp. 43-48).

Brief description of the drawings

The invention is illustrated by drawings, where:

Fig. 1 shows a block diagram of the device;
Fig. 2 shows a block diagram of the capillaroscopy unit;
Fig. 3 shows the operating results of the capillaroscopy unit: (a) selection of the size of the perivascular zone in images of the capillaries; (b) images of the capillaries of a patient with arterial hypertension;
Fig. 4 shows a block diagram of the input unit;
Fig. 5 provides the functioning algorithm of the device;

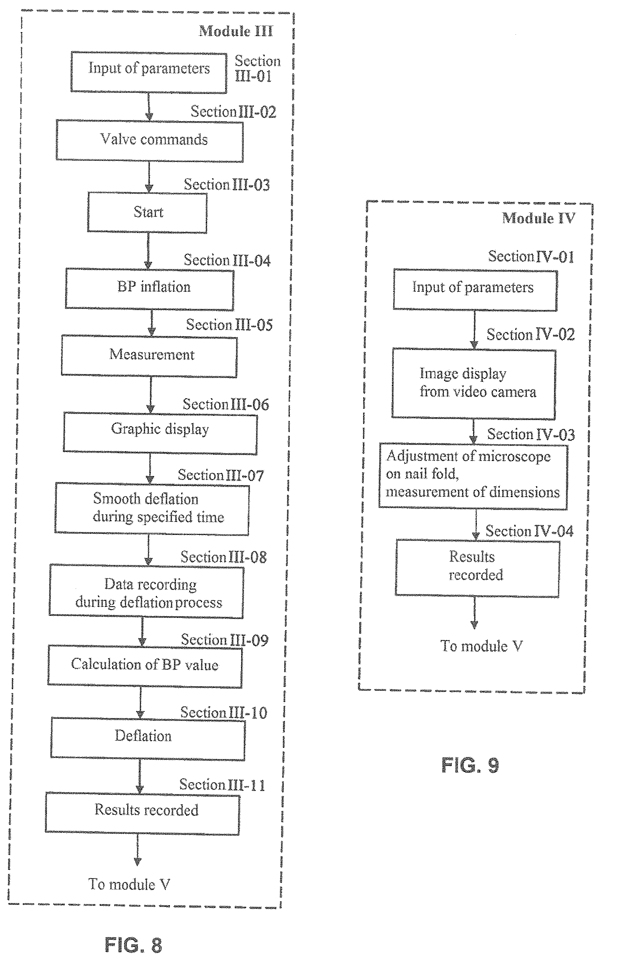

Figs. 6-9 provide the functioning algorithms of certain modules in the process of measurements and calculations for the required values.

Preferred embodiment

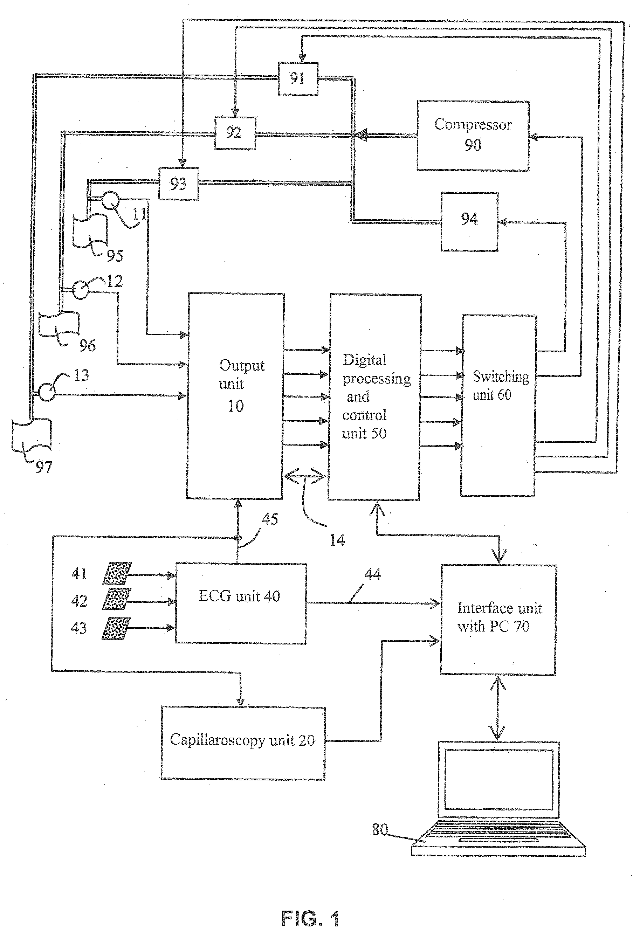

The device (see Fig. 1) contains input unit 10 designed for the reception and adjustment of signal level, amplification, and pre-filtering of the input signals, capillaroscopy unit 20, ECG unit 40, amplitude-to-digital conversion unit 50 and digital signal processing unit 60, switching unit 70 connected to computer 80. Computer 80 provides synchronization of all units, computation of the desired parameters, and data base maintenance.

The pneumatic part makes it possible to inflate and deflate pressure in the occlusive cuffs through commands generated by switching unit 60. Compressor 90 through electro-pneumatic valves 91, 92, and 93 for inflating and electro-pneumatic valve 94 for deflating is connected via air lines to occlusive cuffs 95, 96, and 97 (cuff 95 has the greatest width, cuff 96 has a smaller width, and cuff 97 has the smallest width (mini), on the finger. The control valves 91-94 and the control input of compressor 90 are connected to the control outputs of unit 60. In the air lines of the occlusive cuffs 95, 96 and 97 pressure sensors 11, 12, and 13 are installed and connected to the signal input unit 10.

Capillaroscopy unit 20 is designed to determine blood flow parameters; the design and structure of the unit are discussed below.

ECG unit 40 provides a standard method for signal reception through electrodes 41, 42 and reference electrode 43, generates ECG signals, and transmits them through USB unit 44 in unit 70. ECG unit 40 also generates pulse signal timing linked to the R wave of the ECG, which is fed through bus 45 to the clock inputs of unit 10 and capillaroscopy unit 20, and bus 14 to input unit 50 to designate points of timing relative to signal latency in the capillaries.

Unit 50 converts all incoming analog signals into code, performs digital signal processing, and calculates the necessary parameters, while controlling via unit 70 the entire measurement process through the commands of computer 80.

Switching unit 60 supplies a working voltage to compressor 90 and valves 91-93, providing pumping for cuffs 95, 96, and 97 in accordance with control commands from unit 50. In addition, unit 60 performs signal conversion of pulse width modulation (PWM) signals from unit 50, and DC voltage supply for valve 94, providing smooth cuff deflation.

Unit 70 interface with a computer bundles data flows from units 20, 40, and 60 and provides two-way communication with computer 80.

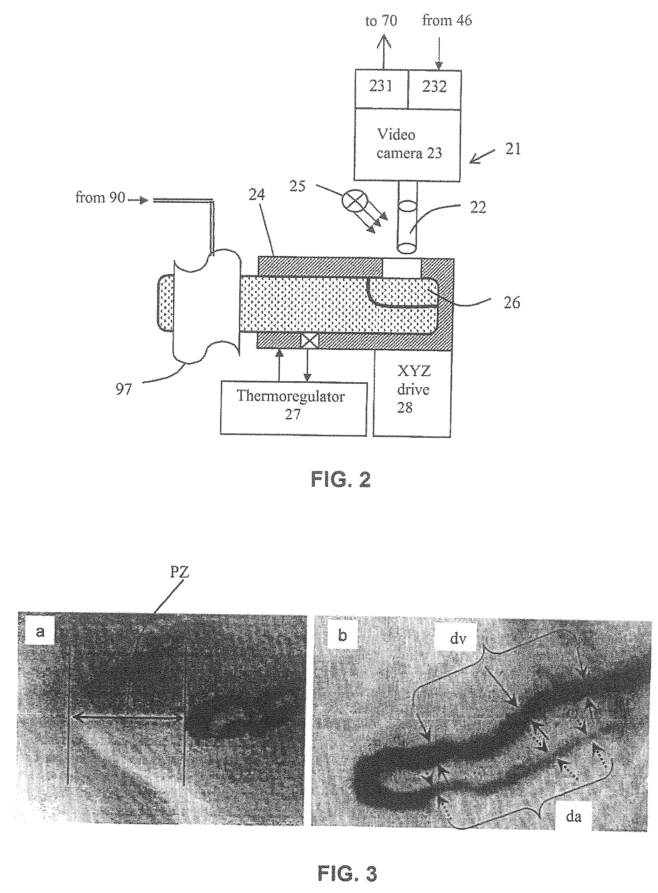

A block diagram of capillaroscopy unit 20 is shown in Fig. 2. Purpose of capillaroscopy unit 20: together with computer software 80 to determine the size of PZ perivascular areas and calculate the capillary remodelling coefficient R on the basis of measured values. In addition, the results of signal analysis in unit 20 are used to calculate PPWV and parameters of the endothelial function. Unit 20 includes optical sensor 21 which runs on the basis of microscope 22, connected to video camera 23. Cradle 24 for brushes made of heat-conducting material, has LED means 25 to highlight the "cold" light area of the nail plate 26 (nail fold) of the finger and means 27 to maintain the temperature of heated bed 24, for example, by using a Peltier element. The design can be similar to the capillaroscopy unit outlined in the above-mentioned patent RU2294689. Optical micro system 22 has two degrees of magnification: x150 and x300-400. Digital cam recorder 23 should provide an assessment of blood flow in the range from 0 to 1000 mps. For fine adjustment in the area of nail plate 26, 3D positioning system 26 is used and placed under the hand cradle. Cradle 24 for the brush can also be one of the electrodes 41 (42) for measuring the ECG signal.

Digital camera 23 contains microcontroller 231, ensuring the removal of signals from the CCD camera and the transmission of signals through unit 70 to computer 80. An additional function of the microcontroller 231 is as follows: frame counter 232 is installed as a program and it resets the counter when a pulse is received from unit 40 through bus 45. The image frames are assigned a number by counter 232. This is necessary for subsequent evaluation in the computer of delay of the image frames relative to the pulse from unit 40, which makes it possible to calculate PPWV in the selected area. The computer program evaluates blood flow velocity in the capillary selected by the operator, and determines the frame number N which corresponds to this maximum speed. Given the period of frames in the video camera and the frame number N, the signal latency in the capillary is calculated relative to the sync pulse from ECG unit 40. Figure 3 shows fragments of typical images obtained using the above-described capillaroscopy unit 2. Figure 3 also shows the specified PZ distance in microns, between the most remote point of the perivascular area and the nearest point of the transitional area of the capillary. Figure 3,b shows narrow arterial sections of capillaries and the relatively wide venous sections of capillary, the diameters of which are determined. Image recognition and selection of informative features are carried out by well-known morphometric methods. In addition, the diameters of the capillary areas are measured in at least three points for each section, and then averaged. Arrows show approximate measurement point diameters of the dv venous (solid lines) and da arterial (dashed line) areas of capillaries in microns, which are used in the subsequent calculation of capillary remodelling R according to the formula R= dv/da.

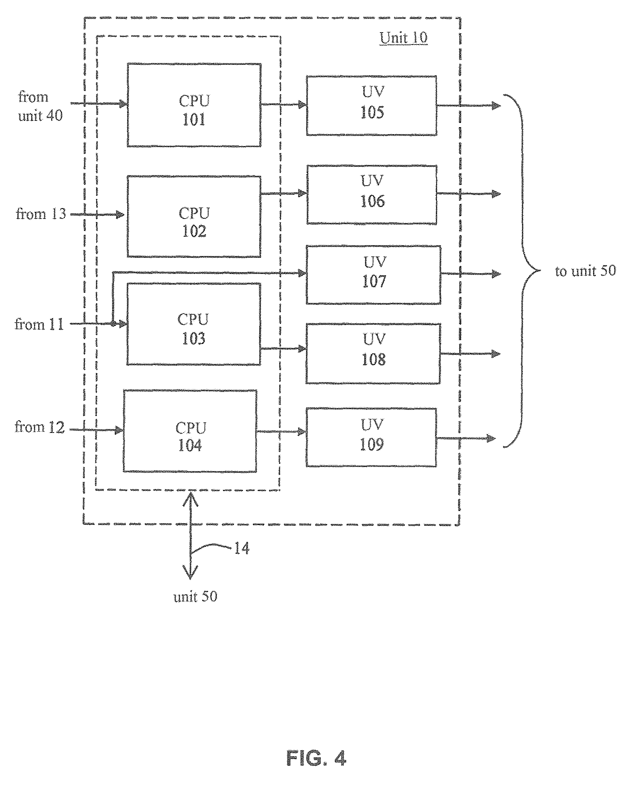

Figure 4 shows a block diagram of input unit 10, which performs the following functions: (1) regulation of the level of signals from pressure sensors 11-13, run by control signals from unit 50, initiated by computer program 80; (2) amplification of signals and their pre-filtering.

Unit 10 includes a line of four digital potentiometers in CPUs 101-104, which are connected through bus 14 to unit 50 and five signal amplifiers (UV) 105-109, equipped with band pass filters designed to suppress interference. The output signal from pressure sensor 11 passes through two circuits: with level control (circuit elements 103 and 108) and directly to the input of amplifier 107. The outputs of all amplifiers 105-109 are connected to the inputs of unit 50 amplitude-to-digital conversion and digital signal processing.

In accordance with the patent-pending method, risk assessment is performed according to the formula

$$K = (PZ + R + PWV + E + BP) \cdot 100, (1),$$

where: PZ = (PZ* - PZm)/ PZσ, where: PZ*, PZm and PZσ are the current value (in microns), the mean and standard deviation of the size of the perivascular zone, respectively;

R = (R*-Rm)/Rσ, where R*, Rm, Rσ is the current value, the mean and standard deviation of the remodelling coefficient, respectively, equal to the ratio (μm) of dv diameters of the venous areas of capillaries to their da arterial areas;

PWV = (PWV*-PWVm)/PWVσ, where PWV*, PWVm, and PWVσ are the current value (in mps), the mean and standard deviation of the velocity of pulse wave propagation, respectively; E = (Em-E*)/Eσ, where E*, Em, Eσ is the current value, the mean and standard deviation of the endothelial function, respectively;

BP = (BP*-BPm)/BPσ, where BP*, BPm, and BPσ are the current value (in mm Hg), the mean and standard values of the mean arterial pressure, which is calculated as the sum of the systolic and diastolic pressure.

The mean value and standard deviation of all parameters included in the formula are defined in the process of experimental research on a group of healthy patients and are then used as constants.

The device is designed to determine the quantities included in the formula (1), and the final calculation of the required K risk indicator.

The device operates as follows.

Before the operation begins, cuffs 95-97 and electrodes 41-43 are placed on the patient's body, preferably according to the table shown below:

| Name of the unit in which the elements function | Item No. | Area of placement (or direction) on the patient's body |
|---|---|---|
| Compressor 90, valves 91-93 | Cuff 95 (greater width) | Shoulder of left arm |
| | Cuff 96 (smaller width) | Wrist of left hand |
| | Cuff 97 (mini) | Phalanx of the ring finger of the left hand |
| Unit 2 capillaroscopy | Hand cradle 24/ microscope 22 | Nail fold of the ring finger of the left hand |
| Unit 4 ECG | Electrode 41 | Wrist of left hand |
| | Electrode 42 | The right wrist/ankle |
| | Electrode 43 | Ankle |

After the device is activated, computer screen 80 displays an image of ECG signals from unit 40. Next the image is adjusted in capillaroscopy unit 20. All measurements are made under the control of computer 80, which responds to the operator's commands. Before measurements are made data of the patient are entered into the computer database. The distance between the centres of cuffs 15, 16, and 17, and the distance from the heart to the cuffs are measured and entered in the database, and are also used in subsequent calculations.

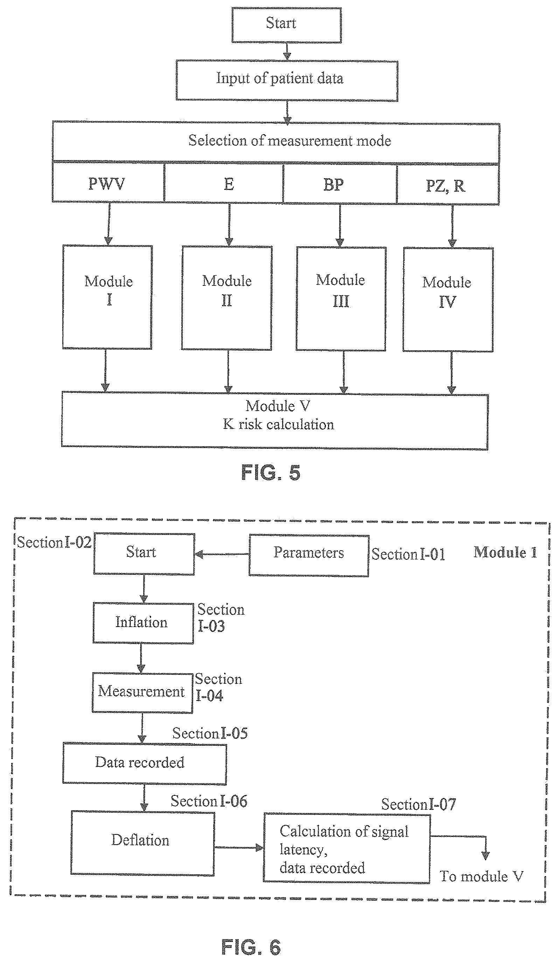

The functioning algorithm of the device as a whole is shown in Fig. 5. The device provides for input of data about the patient, the choice of measurement mode, the functioning of the measuring modules (I-IV) and module V for the calculation of K risk.

Measurement of PWV values (the velocity of pulse wave propagation) (Module I, Fig. 6). The measurement may be performed on areas of shoulder - wrist and heart - capillaries of the finger, and is determined by the placement of cuffs 95-97 and the corresponding pressure sensors 11-13. When initiating PPWV measurement mode (Fig. 6) using computer keyboard 80 and regulators on the window interface, parameters are set - the inflation level of cuffs 95-97 and the length of measurements (section 1-01). Next, the PPWV mode is initiated using the Start button (section I-02) of the program. This opens valves 91-93, while smooth deflation valve 94 is closed. All cuffs 15-17 are inflated to the specified level (section I-03) and all valves (91, 92, and 93) are closed. Next, the actual measurement process (section I-04) is carried out: the program calculates the amplitude signals coming from pressure sensors 11, 12 and 13, and, while adjusting the attenuation in digital potentiometers 102, 103 and 104 of unit 10, sets the nominal signal level and produces signals to be displayed on the screen interface of computer 80.

After that, for a duration set by the operator (see section 1-01) the arrival time of signals is determined and the relative signal latency is calculated in the previously described pairs of signals: shoulder - wrist, heart - capillaries of the finger, and the results are recorded. The obtained results are stored in memory (section 1-05).

Upon expiration of the duration set by the operator for the accumulation of signals from sensors 11-13, all valves (91, 92, and 93) are opened and cuff pressure is released (section I-07). The obtained latency values between the signals are averaged and PPWV (PWV) values are calculated for the various areas according to the formula: PWV = L/ΔT, where L is the distance, ΔT is the signal latency, respectively, between the relevant pair of sensors comprising sensors 11-13, and the ECG (R wave of ECG correlates in a known manner with the opening of the semi-lunar valves of the heart) (section I-07). The recorded data and PWV values are transferred to module V for calculation of the K index.

To measure PWPV using capillaroscopy unit 20 a computer program evaluates the blood flow velocity in the capillary selected by the operator and determines the frame number N which corresponds to this maximum speed. Given the period of frames of the video camera and the frame number N, the signal latency is calculated in the capillary relative to the synchronization pulse from ECG unit 40. This is necessary for subsequent computer evaluation of the image frame delay relative to the momentum of unit 40, thus making it possible to calculate PWPV in the selected area.

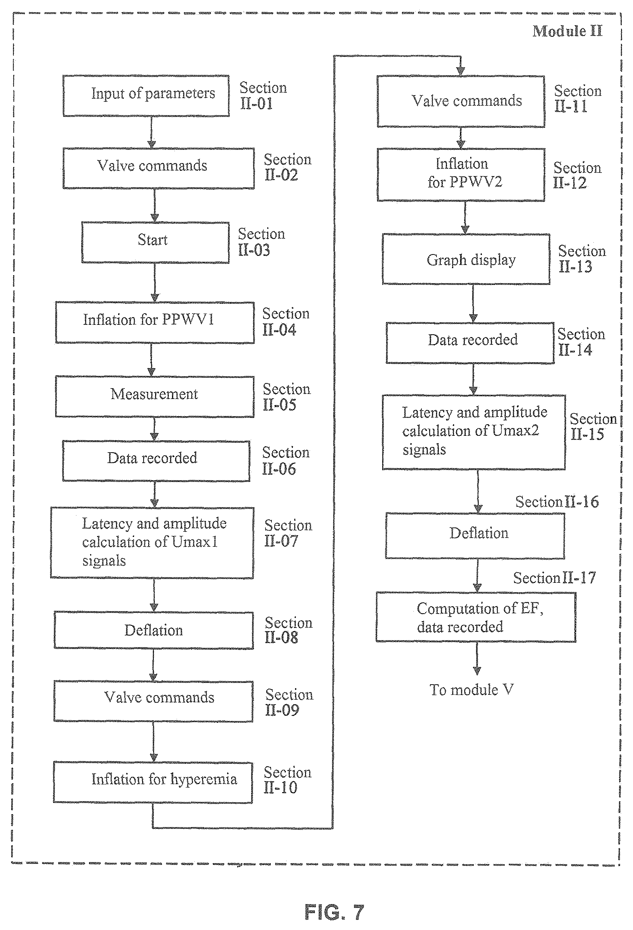

Determination of the endothelial function (EF) (Module II, Figure 7). The EF can be measured in three areas: shoulder-wrist, wrist-finger phalanx, finger phalanx-capillaries, but for practical purposes it is sufficient to know the E index, obtained from the shoulder-wrist plot. In step (section II-01) the following parameters are entered: inflation level of cuffs 95, 96 or 97 for the selected area (for example, on the shoulder or finger) in PPWV measurement mode, under clamping, and the duration of the measurements. The commands are transmitted to valves 91, 92 and 93, which are open, while valve 94 is closed (section II-02). Next, start EF measurement mode using the Start button (section II-03) of the program (section II-04).

PPWV measurement mode is activated. The device measures the latency and amplitude of U1 signals from pressure sensors, displays charts of signals, and automatically adjusts the signal levels (section II-05). Data is recorded (section II-06) on the measurement time of PPWV. The latency and amplitude of Umax1 are calculated based on the results of the measurements (section II-07) then the pressure is released (section II-08).

The valve connected to the selected cuff is opened (for example, cuff 95 and valve 93), while the other valves (91, 92) are closed (section II-09). The selected cuff is inflated to the proper level of blood flow restriction (section II-10). All valves are closed (II-11). The program maintains a specified restriction time - three minutes are sufficient, periodically inflating the cuff to the required level (section II-12), to compensate for deflation in the pneumatic system, if necessary. All graphs of signals are displayed on the computer screen (section II-13) and the data are recorded (section II-14). Then all the valves are opened, pressure in the selected cuff drops almost to zero, then a pause is observed as specified for this state.

The PPWV measurement mode is reactivated - measuring the amplitude of U2 signals from the pressure sensors and calculating the maximum amplitude value of Umax2 (section II-15). Signal amplitude algorithms are not adjusted during the repeat measurements. Next, the pressure is released (section II-16) and the E index is calculated as the ratio of the signals measured as a result of blood flow restriction and without it, according to the formula E= Umax2/Umax1 (section II-17).

The image from camera 23, the signals from sensors 11-13, and all measurement results are displayed on the computer screen. The data obtained are entered in the database.

Measurement of the arterial pressure (BP) index. Measurements are performed in unit 10 using shoulder cuff 95 and pressure sensor 11. BP measurement mode is activated, input of parameters is performed (section III-01, the level is set for inflating the cuffs to measure BP, the time of measurement, and valve commands are generated (section III-02). BP measurement mode is initiated by pressing the Start button (section III-03) of the program.

The computer program executes the following algorithm: valve 93, connected to cuff 95 is opened, while the other valves are closed. Cuff 95 is inflated to a specified level (section III-04). All valves are closed. The graphs are displayed (section III-05). Valve 94 is opened with a smooth release of pressure. Unit 60 controls valve 94 in such a way that a linear decrease in pressure takes place in cuff 95 at a given speed (section III-06). Simultaneously, the computer records signal pulses from pressure sensor 11, which is connected to cuff 95 (section III-07). After the pressure has been deflated, the stored signals (section III-08) are analyzed by the computer program using algorithms adopted for the oscillometric method of BP measurement (see, for example, RU2343826). Next, the pressure is released (section III-9). The BP parameters are measured: systolic and diastolic pressure. The measurement is performed three times at a specified interval. The image signals from sensor 11 and all measurement results are displayed on the screen (section III-10) and entered in the database.

The required risk index K of cardiovascular complications is calculated on computer 80 on the basis of the current values obtained for the parameters according to the above-mentioned formula (1): K = (PZ + R + PWV + E + BP) · 100. The mean value and standard deviation of all parameters are determined in the process of experimental research on a group of healthy patients and then used as constants.

The numerical values of the K index value lie in a range from 1 to 1000.

Experimental validation. The ability to meet the technical result with an increase in the reliability assessment of the risk of cardiovascular complications is confirmed by the results from testing the method in patients with type 2 diabetes mellitus and patients with type 2 diabetes mellitus complicated by arterial hypertension.

Measurements were made on a group of healthy volunteers (40 years and above, average age of 51.5±6.1; n = 45), patients with type 2 diabetes mellitus (average age of 48.9±9.6; n = 32), and patients with type 2 diabetes mellitus complicated by arterial hypertension (average age 47.4±14.7 n=29). The numerical values are summarized in the Table.

The groups of patients were matched by age with the group of healthy volunteers.

The results of the numerical evaluation according to the formula (1) yield the following results:

$$K_1 = (110.5 - 102.2): 12,2 + (1.53 - 1.3): 0.1 + (9.0 - 7.8): 1.0 + (73.4-57.9):34.5 + + (94.7-91.3): 9.5 = 4.99 \cdot 100 = 499.$$

$$K_2 = (115.3 - 102.2):12.2 + (1.60 - 1.35): 0.1 + (9.2 - 7.8):1.0 + (73.4 - 40.5):34.5+ + (103.5 - 91.3):9.5 = 7.2 \cdot 100 = 720.$$

The above-mentioned clinical studies were used to identify risk criteria for cardiovascular complications in patients with type 2 diabetes mellitus and patients with type 2 diabetes mellitus complicated by arterial hypertension.

It was established that at K index values ranging from 1 to 249, the risk of cardiovascular complications is assessed as low, in a range from 251 to 499 there is an intermediate risk, in a range from 500-749 there is a high risk, and in a range from 750-100 there is a very high risk.

Table

| RESULTS OF MEASUREMENT ON GROUPS OF HEALTHY VOLUNTEERS AND PATIENTS WITH DIABETES MELLITUS | | | | | | | | | | |
|---|---|---|---|---|---|---|---|---|---|---|
| Subjects | | | Recorded values | | | | | | | |
| Features | Average age | M, σ | PZ | R | PWV | E | BPs | BPd | BPamv |
| Healthy volunteers (40 years and above) | 51.6±6.1 (n=45) | m | 102.2 | 1.3 | 7.8 | 73.4 | 113.0 | 69.5 | 91.3 |
| | | σ | 12.2 | 0.1 | 1.0 | 34.5 | 11.2 | 7.7 | 9.5 |
| Patients with type 2 diabetes mellitus | 48.9±9.6 (n=32) | m | 110.5 | 1.53 | 9.0 | 57.9 | 115.3 | 74 | 94.7 |
| | | σ | 3.7 | 0.2 | 1.3 | 35.2 | 9.1 | 7.8 | 8.5 |
| Patients with type 2 diabetes mellitus + arterial hypertension | 47.4±14.7 (n=29) | m | 115.3 | 1.6 | 9.2 | 40.5 | 129.9 | 77.0 | 103.5 |
| | | σ | 4.0 | 0.3 | 2.0 | 0.22 | 25.7 | 15.8 | 20.8 |

It is important to note that in contrast to the existing methods for assessing the risk of cardiovascular complications, based on historical, instrumental and laboratory data, the patent-pending method during one stage of research makes it possible to quantitatively evaluate the current degree of impairment of the entire cardiovascular system beginning with the state of the myocardium, main arteries down to the smallest arteries, including capillaries and through which the exchange between tissues and blood occurs.

From comprehensive studies carried out to assess the risk of cardiovascular complications, only the most significant indicators are selected, i.e. those which to the greatest extent reflect in modern medicine the presence of pathological changes and, accordingly, the risk of cardiovascular complications.

Industrial applicability

The patent-pending method and device offer a new criterion - a quantitative risk assessment index for cardiovascular complications, thus allowing the patient, through repeated contacts and a study that is carried out, to assess the effectiveness of the prescribed treatment.

**Claims**

1. The method for assessing the risk of cardiovascular complications includes the following operations:

   determining the size of the perivascular zone, the diameters of venous and arterial areas of capillaries through nailfold capillaroscopy of the hand;
   determining the values of propagation of pulse wave velocity and the endothelial function in the upper limb synchronously relative to the R peak of the ECG, and blood pressure measurement;
   calculating the K index on the risk of cardiovascular complications using the formula:

$$K = (PZ + R + PWV + E + BP) \cdot 100,$$

   where: $PZ = (PZ^*-PZm)/PZ\sigma$; where: $PZ^*$, $PZM$, $PZ\sigma$ is the current value, the mean and standard deviation of the size of the perivascular zone, in microns, respectively;
   $R = (R^*-Rm)/R\sigma$; where: $R^*$, $RM$, $R\sigma$ is the current value, the mean and standard deviation of the capillary remodelling coefficient;
   $PWV=(PWV^*-PWVm)/PWV\sigma$, where: $PWV^*$, $PWVm$, $PWV\sigma$ is the current value, the mean and standard deviation of the propagation of pulse wave velocity, mps, respectively;
   $E = (EM-E^*)/E\sigma$. where: $E^*$, $Em$, $E\sigma$ is the current value, the mean and standard deviation of the endothelial function, respectively;
   $BP = (BP^*-BPm)/BP\sigma$, where: $BP^*$, $BPm$, $BP\sigma$ is the current value, the mean and standard deviation of the half-sum of systolic and diastolic blood pressure, and mm Hg, respectively, with the mean values and standard deviations of all parameters included in the formula obtained from the study of healthy patients;

   the risk of cardiovascular complications at a K index value in the range of 1-249 is categorized as low risk, a K index value in the range of 250-499 as intermediate risk, a K index value in the range of 500-749 as high risk, and a K index value in the range of 750-1000 as a very high risk.

2. The method according to claim 1, **characterized in that** the current value of the R* capillary remodelling coefficient is determined by the formula R*= dv/da, where dv, da is the diameter of the venous and arterial areas of capillaries, microns, respectively.

3. The method according to claim 1, **characterized in that** the current value PZ* for the size of the perivascular zone is defined as the distance between the most remote point of the perivascular zone and the nearest point of the transitional area of a capillary.

4. The method according to claim 1, **characterized in that** the propagation of the pulse wave velocity is determined in the should-wrist area by recording the pressure sensor signals which correspond to the time the pulse wave takes to pass during occlusion, with subsequent averaging and accumulation of the above-mentioned signals over a specified time measurement interval and the PWV value for this specific section is calculated according to the formula: PWPV*= L/$\Delta$T mps, where L and $\Delta$T are the distance and signal latency between the pressure sensors, respectively.

5. The method according to claim 1, **characterized in that** the current value of the E* index is determined by the results of measuring the amplitude U1 and U2 signals of the pressure sensors in the same area of the shoulder-wrist, at least twice, before and after the test with compression of the brachial artery, preferably three minutes, averaging and accumulation of signals within a specified time measurement interval, and the indicator is calculated according to the formula E*= Umax2/Umax1, where Umax1 and Umax2 are the maximum values of the signal amplitude of the pressure sensors before and after the test, respectively.

6. The device for assessing the risk of cardiovascular complications, including:

   pneumatic facilities for creating occlusion on the limb of the subject, containing occlusive cuffs, connected through electro-pneumatic valves to a compressor and an electro-pneumatic valve with smooth deflation;
   pressure sensors connected to occlusive cuffs;

the input unit has the ability to receive control signals from the output of the pressure sensors, amplification and pre-filtering;

the optical capillaroscopy unit has the ability to determine the size of the perivascular zone, and the diameters of the venous and arterial areas of the capillaries of the nail fold of the hand under reactive hyperaemia and in its absence;

the electrocardiogram recorder has the ability to generate a pulse synchronization signal on the R wave of the electrocardiogram;

the analog-to-digital conversion and digital signal processing unit, the input of which is connected to the output of the above-mentioned input unit and the output synchronization of the above-mentioned electrocardiogram recorder, which has the ability to average and accumulate signals within specified time interval measurements, as well as determine their amplitude and time parameters;

the switching unit, the input of which is connected to the output of the above-mentioned analog-to-digital conversion and digital signal processing unit, and the output to the inputs of the above-mentioned electro-pneumatic valves and a compressor;

the computer interface system, the inputs and outputs of which are connected to the amplitude-to-digital conversion and signal processing unit, and to a computer, and the inputs are connected to the outputs of the electrocardiogram recorder and the optical capillaroscopy unit, the input of which is connected to the output clock of the above-mentioned electrocardiogram recorder.

7. The device according to claim 6, in which the input unit has four digital potentiometers, the signal inputs of which are the inputs of the unit, and five amplifier signals equipped with band pass filters, the outputs of which are the outputs of the unit, in which

the digital outputs of the potentiometers are connected to the inputs of the four amplifiers in series, and the fifth input of the amplifier is connected directly to the input of one of the digital potentiometers.

8. The device according to claim 6, in which the optical capillaroscopy unit contains a hand cradle to brush the upper limb, made of heat-conducting material connected to the cradle for highlighting areas of the nail plate of the finger extremity, an occlusive finger cuff, a tool for maintaining the temperature of the heated bed, an optical sensor based on a microscope with a video camera, a microcontroller connected to the video camera, which is the output of the optical capillaroscopy unit, a 3D positioning system for the cradle relative to the optical sensor, which can be configured to adjust the image area of the nail plate.

9. The device according to claim 8, in which the cradle performs the function of one of the electrodes of the electrocardiogram recorder.

10. The device according to claim 8, in which the microcontroller contains an image frame counter which assigns a sequential number, with the ability to reset the counter of the synchronization signal on the R wave of the electrocardiogram.

FIG. 1

FIG. 2

FIG. 3

Unit 10

from
unit 40 → CPU
101 → UV
105 →

from 13 → CPU
102 → UV
106 →

UV
107 →

from 11 → CPU
103 → UV
108 →

from 12 → CPU
104 → UV
109 →

to unit 50

14

unit 50

FIG. 4

```
                        ┌─────────────┐
                        │    Start    │
                        └─────────────┘
                               │
                               ▼
                    ┌────────────────────┐
                    │ Input of patient data │
                    └────────────────────┘
                               │
                               ▼
    ┌──────────────────────────────────────────────────┐
    │          Selection of measurement mode           │
    ├──────────┬──────────┬──────────┬─────────────────┤
    │   PWV    │    E     │    BP    │     PZ, R       │
    └──────────┴──────────┴──────────┴─────────────────┘
         │          │          │          │
         ▼          ▼          ▼          ▼
    ┌────────┐  ┌────────┐  ┌────────┐  ┌────────┐
    │ Module │  │ Module │  │ Module │  │ Module │
    │   I    │  │   II   │  │  III   │  │   IV   │
    └────────┘  └────────┘  └────────┘  └────────┘
         │          │          │          │
         ▼          ▼          ▼          ▼
    ┌──────────────────────────────────────────────────┐
    │                   Module V                        │
    │              K risk calculation                   │
    └──────────────────────────────────────────────────┘
```

**FIG. 5**

Section I-02   Start  ◄── Parameters   Section I-01   **Module 1**

Inflation   Section I-03

Measurement   Section I-04

Section I-05   Data recorded

Section I-06   Deflation → Calculation of signal latency, data recorded   Section I-07   To module V

**FIG. 6**

15

FIG. 7

**Module III**

Input of parameters — Section III-01

↓

Section III-02
Valve commands

↓

Section III-03
Start

↓

Section III-04
BP inflation

↓

Section III-05
Measurement

↓

Section III-06
Graphic display

↓

Section III-07
Smooth deflation during specified time

↓

Section III-08
Data recording during deflation process

↓

Section III-09
Calculation of BP value

↓

Section III-10
Deflation

↓

Section III-11
Results recorded

↓

To module V

**FIG. 8**

**Module IV**

Section IV-01
Input of parameters

↓

Section IV-02
Image display from video camera

↓

Section IV-03
Adjustment of microscope on nail fold, measurement of dimensions

↓

Section IV-04
Results recorded

↓

To module V

**FIG. 9**

<div align="center">INTERNATIONAL SEARCH REPORT</div>

| International application No. |
|---|
| PCT/RU 2013/001083 |

**A.    CLASSIFICATION OF SUBJECT MATTER**

*A61B 5/02 (2006.01)*      *A61B 5/145 (2006.01)*
*A61B 5/04 (2006.01)*      *A61B 10/00 (2006.01)*
                                          *G06F 19/00 (2011.01)*

According to International Patent Classification (IPC) or to both national classification and IPC

**B.    FIELDS SEARCHED**

Minimum documentation searched (classification system followed by classification symbols)

<div align="center">A61B 5/02, 5/04, 5/145, 10/00, G06F 19/00</div>

Documentation searched other than minimum documentation to the extent that such documents are included in the fields searched

Electronic data base consulted during the international search (name of data base and, where practicable, search terms used)

<div align="center">"Российская медицина", EAPATIS, Espacenet, Medline, PAJ, Patentscope, PatSearch (RUPTO internal), RUPTO, Rambler, USPTO, Yandex, Benran, ВИНИТИ, РМЖ</div>

**C.    DOCUMENTS CONSIDERED TO BE RELEVANT**

| Category* | Citation of document, with indication, where appropriate, of the relevant passages | Relevant to claim No. |
|---|---|---|
| D, A | RU 2352258 C2 (GOSUDARSTVENNY NAUCHNO-ISSLEDOVATELSKY TSENTR PROFILAKTICHESKOI MEDITSINY et al.) 20.04.2009, the claims | 1-5 |
| D, Y<br>A | US 2006/0264771 A1 (DAILYCARE BIOMEDICAL INC.) 23.11.2006, paragraphs [0015], [0016], [0047] - [0050], fig. 5 | 6, 8<br>7, 9, 10 |
| D, Y | RU 2343826 C1 (OBSHCHESTVO S OGRANICHENNOI OTVETSTVENNOSTJU "AKTUALNYE MEDITSINSKIE DIAGNOSTICHESKIE TEKHNOLOGII") 20.01.2009, abstract, the claims, fig. 2 | 6, 8 |
| D, Y | RU 2294689 C2 (GURFINKEL JURY ILICH) 10.03.2007, p. 4-6, fig. 1 | 6, 8 |

☐    Further documents are listed in the continuation of Box C.          ☐    See patent family annex.

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 10 April 2014 (10.04.2014) | 07 May 2014 (07.05.2014) |

| Name and mailing address of the ISA/ | Authorized officer |
|---|---|
| RU | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2410017 C1, Khoronenko
- RU 2342068 C2
- RU 2405418 C1, Podtaev
- RU 2389434 C2, Gurfinkel
- RU 2352258 C2, Oganov
- RU 2343826 C1, Gurfinkel
- RU 2373846 C1, Pegov

- US 6939304 B2, SCHNALL ROBERT
- RU 2294689 C2, Walejko
- WO 9804182 A2, GOOR DANIEL A
- US 2006224073 A1, LIN, KANG-PING
- US 2006264771 A1, LIN KANG-PING
- RU 2294689
- RU 2343826

**Non-patent literature cited in the description**

- **GURFINKEL YU.I. ; MAKEEVA, O.V. ; OSTRO-ZHINSKY V.A.** Features of microcirculation, the endothelial function, and the propagation of pulse wave velocity in patients with initial stages of hypertension. *Functional diagnostics,* 2010, 18-25
- **GURFINKEL YU.I. ; KATZ N.V. ; PARFENOVA L.M. ; IVANOVA I.YU. ; ORLOV V.A.** Comparative study of the propagation velocity of the pulse wave and endothelial function in healthy individuals and patients with cardiovascular pathology. *Russian Journal of Cardiology,* 2009, 38-43

- **GURFINKEL YU.I. ; KUDUTKINA M.L ; PARFENOVA L.M. ; ORLOVA V.A.** Features of microcirculation in patients with chronic heart failure against the background of treatment with ACE inhibitors and diuretics. *Russian Journal of Cardiology,* 2011, 43-48